# EUROPEAN PATENT APPLICATION

(11) **EP 2 050 810 A2**
(43) Date of publication of application: **22.04.2009**
(21) Application number: 08253369.6
(22) Date of filing: 17.10.2008
(51) Int. Cl.: C10L 1/23, C10L 10/12, C07C 201/02, C07C 203/04

(54) **Method for production of cetane-index improvement additive for diesel oil**

(30) Priority: 18.10.2007 BR PI0703899
(71) Applicant: Petroleo Brasileiro S.A. Petrobras, Rio de Janeiro, RJ (BR)
(72) Inventor: Rabello, Carlos Rene Klotz, Rio de Janeiro, RJ - CEP: 22.631-051 (BR); Siqueira, Bernardo Galvâo, Rio de Janeiro, RJ - CEP: 22.631-051 (BR); De Menezes, Raphael Bezerra, Rio de Janeiro, RJ - CEP: 21.330-270 (BR)
(74) Representative: Benson, John Everett

(57) **Abstract**

A method is presented for production of a cetane-index improvement additive for diesel oil produced from biodiesel from castor bean oil.

Said method employs nitration at the site of the hydroxyl functionality already forming part of the natural molecular chemical structure of the fatty acid ester generated in the production of biodiesel obtained from castor bean oil.

The present method is advantageous through having a lower number of processing stages, lower rate of undesired nitration of unsaturations, lower consumption of nitrating mixture, reduction in the level of attachment of NO₂ to the molecules of the additive and consequent reduction in undesirable environmental impacts caused by NOₓ, and utilising a raw material from a renewable source.

The present cetane improvement additive may be employed as an additive to diesel oil of mineral origin, biodiesel, or synergetic mixtures thereof in any proportion.

## Description

### Field of the invention

The present invention has as its objective a method for production of a cetane-index improvement additive for diesel oil.

More precisely the present invention relates to a method for production of a cetane-index improvement additive for diesel oil through nitration of the molecule of fatty acid ester generated in the production of biodiesel.

More specifically the present invention relates to a method for production of a cetane-index improvement additive for diesel oil through direct nitration at the site of the hydroxyl functionality already present in the natural chemistry of the molecule of the fatty acid ester generated in the production of biodiesel produced from castor bean oil.

### Background of the invention

The employment of cetane-index improvement additives is necessary to make refinery-produced diesel oil suitable for modern engines, with the objective of improving the performance thereof.

Nitrates of medium- to long-chain hydrocarbons are known to be effective as cetane-index improvers.

An important compound utilised as a cetane-index improvement additive for diesel fuels is 2-ethylhexyl nitrate (2-EHN) obtained from nitration of 2-ethylhexanol.

The technological route most utilised for production of 2-ethylhexanol employs propene as raw material, being subjected to a hydroformylation process (olefin reaction with CO and H₂) to lengthen the chain, in this manner generating iso-butanal and n-butanal. Two molecules of n-butanal are subsequently condensed obtaining 2-ethylhexanal, this being subjected to a hydrogenation process to obtain 2-ethylhexanol.

In general cetane improvers are obtained from nitration of alcohols and glycols, such as the aforesaid 2-ethylhexanol and ethylene glycol.

New proposals may be observed in the specialised technical literature for the production of cetane-index improvement additives such as nitration of the unsaturations contained in the molecules of biodiesel obtained from, not however being limited to, oils of vegetable origin such as, for example, those of sunflower and of olive, as well as others.

However the nitration of such compounds not being selective it leads to exaggerated consumption of nitric acid because, in addition to acting on the double bonds existing in the molecules of said fatty acids, it also takes place at saturated carbons.

In addition, there has not been found any publication including direct nitration at the site of the hydroxyl already present in the natural molecular chemical structure of the fatty acid ester, as in the case of biodiesel from castor beans, utilised as consumable in the present invention.

Thus an objective of the present invention is to utilise said fatty ester derived from castor bean oil, being already hydroxylated due to its chemical nature, as raw material for subjection to nitration at the site of the hydroxyl for the production of a nitrated cetane-index improvement additive.

### Related art

Nitrated cetane-index improvement additives are produced from higher alcohols such as 2-ethylhexanol (2-EH) and its subsequent nitration for the obtainment of the additive 2-ethylhexyl nitrate (2-EHN).

In this manner, in known methods for the production of nitrated cetane improvement additives there exists a requirement for the synthesis of higher alcohols, the production costs whereof are very high by virtue of involving a technological route of great complexity.

Another possibility for production of cetane improvement additives is the utilisation as raw material of biodiesel obtained from triglycerides, their being subsequently nitrated on their double bonds. However such nitration is not very selective leading to excessive consumption of reagents.

United States patent US 5 454 842 (Poirier), introduced herein for reference, presents three different methods for production of nitrates from supplies of triglycerides.
- The first of such methods includes:
   1) hydrolysis of triglycerides to fatty acids;
   2) esterification of such fatty acids with a diol such as ethylene glycol; and
   3) nitration of the primary alcohol group of the glycol ester of the fatty acid.

Nitration is carried out using nitric acid in combination with a strong acid. This first method is focused on total transesterification, producing primary alcohols.
- The second method described by Poirier includes:
   1) hydrolysis of triglycerides to fatty acids;
   2) hydration of the double bonds of the fatty acid chain by a reaction catalysed by formic acid and reaction with hydrogen peroxide; and
   3) nitration of the secondary alcohol groups.
- The third method described by Poirier includes:
   1) hydrolysis of triglycerides to fatty acids;
   2) hydrogenation of the double bonds of the fatty acid chain;
   3) esterification of such acids with methanol;
   4) reduction of the ester link to a primary alcohol; and
   5) nitration of the alcohols.

It may be observed that in Poirier's publication there exists the requirement to produce primary and/or secondary alcohol groups in order to then carry out the nitration. However the production of higher alcohols as a condition for realising such nitration increases the number of processing stages and the respective installations and equipment to satisfy the parameters involved in the processes comprising such method, together with excessive consumption of reagents, increasing the final cost of the additive.

United States patent application US 2001/0037598 (Suppes, Galen J., et al), introduced herein for reference, presents a method for converting triglycerides into cetane improvement additives containing hydrocarbon and nitrate groups which, in general terms, includes direct nitration of the double bonds to form nitrates.

In the case of United States patent US 2001/0037598 the fatty acid ester is derived from a naturally-occurring triglyceride and nitration is achieved through hydration of at least one double bond of the fatty acid and subsequent nitration of the secondary alcohols thus produced. Consequently nitration of such secondary alcohols produced by hydration of at least one double bond of the fatty acid directly on the double bonds causes an increase in consumption of the nitrating mixture contributing to the addition of another portion of the processing cost.

The costs involved in the aforementioned procedures for obtainment of the cetane improvement additive, which in its manufacture utilises normal methods involving costly and difficult processes, affect the subsequent phases of adjustment of the cetane index of the diesel oil produced in refineries, and have a direct effect on the price of the final fuel product.

To date the publications referred to in the state of the art have the objective of producing cetane improvement additives from triglycerides involving a series of industrial procedures which, among other costs, add those of the equipment and consequent reaction-controlling devices for the obtainment of the compound of interest for the final process of nitration. In addition thereto, breaking double bonds during the nitration procedures in the aforementioned existing art may cause splitting of the biodiesel molecule, leading to a loss of yield from the process or production of a fuel having a large number of NO₂ molecules attached to the chain.

A further aspect to be additionally considered is that in some countries the additive 2-EHN (commercial) requires to be imported adding an even larger portion of cost to affect the additive-containing diesel thus produced and, consequently, the production of an additive replacing such imported 2-EHN, of low cost, is very attractive.

In terms of the environmental aspect the aforementioned methods, already in existence in the art, involve processes leading to nitration not restricted to sites carrying the hydroxyl functionality characteristic of the primary and/or secondary alcohol function. Such nitration also occurs through breaking double bonds existing in the chain, increasing consumption of the nitrating mixture and producing as a result an additive leading to a final fuel having a high number of NO₂ molecules attached to the chain of the raw material employed.

Consequently the excessive increase in NO₂ attachment to the fatty ester molecule of the additive, in addition to increasing consumption of the nitrating mixture, is problematic and should be avoided at all costs due to the fact that, following combustion, said NO₂ group is converted into NOₓ in the atmosphere, having further undesirable effects on the environment such as attack on the ozone layer, in addition to being harmful for human beings, causing irritation of mucous membranes, and increasing the probability of the occurrence of the phenomenon called "acid rain".

As a consequence there exists a requirement for the provision of a method for the obtainment of a nitrated additive having an operational efficacy similar to 2-EHN, produced in a more simplified manner with lower costs, yet having easy logistics, that is to say producible within the very installation wherein biodiesel is produced and which, in addition, is not aggressive towards the environment.

The present invention solves the aforementioned matters, providing an advantageous method for production of a nitrated cetane improvement additive, employing as raw material biodiesel produced from castor bean oil.

### Summary of the invention

The present invention describes a method for producing a cetane-index improvement additive for diesel oil by nitration carried out at the site of the hydroxyl functionality already present in the natural molecular chemical structure of the chains of fatty esters obtained from castor bean biodiesel, the principal characteristic whereof being the use of such fatty ester derived from castor bean oil, which due to its chemical nature is already hydroxylated, as raw material to be subjected to direct nitration at the site of the hydroxyl for production of said nitrated cetane improvement additive.

As is known, cetane number correlates well with ignition timing retard and in this manner the products of the present invention may be used with diesel or related fuels employed in compression-ignition engines leading to advantages in performance associated with the utilisation there of with diesel fuels, having reduced emissions to the environment and offering easier cold starting.

The present invention is a method overcoming the disadvantages of existing methods in the art, delineated above, and constitutes an improved nitration process resulting in:

| | |
|---|---|
| 1 | lower number of processing stages; |
| 2 | lower rate of undesired nitration of unsaturations; |
| 3 | lower consumption of reagents; |
| 4 | improved performance in relation to the nitrogen content of the final product in terms of its environmental impact; and |
| 5 | use of raw material from a renewable source. |

### Brief description of the figures

Figure 1 is a block diagram representing the stages of the process of nitration of hydroxylated biodiesel produced from castor bean oil.

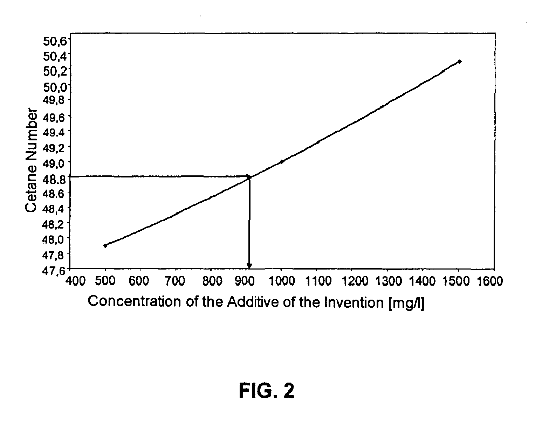

Figure 2 shows a graph of concentration of such nitrated castor bean diesel oil additive against variation in cetane index, utilised to compare the performance thereof in relation to commercial 2-ethylhexyl nitrate.

### Detailed description of the invention

In the present application the term "diesel oil" to be treated with said cetane improvement additive, unless specifically defined, comprises diesel oil of mineral origin, biodiesel oil, or synergetic mixtures of both thereof in any proportion.

The terms "hydroxylated biodiesel", "castor bean biodiesel", "hydroxylated fatty ester derived from castor bean oil", unless specifically defined, comprise biodiesel formed from processing castor bean oil already possessing in the natural chemistry thereof a hydroxyl group in its molecular chain.

An important characteristic which castor bean biodiesel presents is that the fatty acid ester thereof already possesses in its natural chemistry a hydroxyl functionality in its molecule permitting that it may serve as primary consumable, being sent directly to the nitration stages, avoiding costs involved in the intermediate production of the alcohol, as occurs in the production of 2-ethylhexyl (2-EH) and also in other methods already in existence in the art.

Nitration carried out directly at the site carrying the hydroxyl functionality of the molecule of hydroxylated fatty ester of castor bean biodiesel makes greater selectivity possible, reducing indiscriminate nitration of double bonds existing in the molecule, avoiding the disadvantages of methods already in existence in the art, as aforesaid.

Increase in the cetane index may occur through addition of the additive of the present invention to the stream of biodiesel itself or through addition thereof to a stream of diesel derived from petroleum.

The processes involved in the production of castor bean biodiesel, used as starting raw material for the present invention, are already known and consequently shall not be given in detail herein.

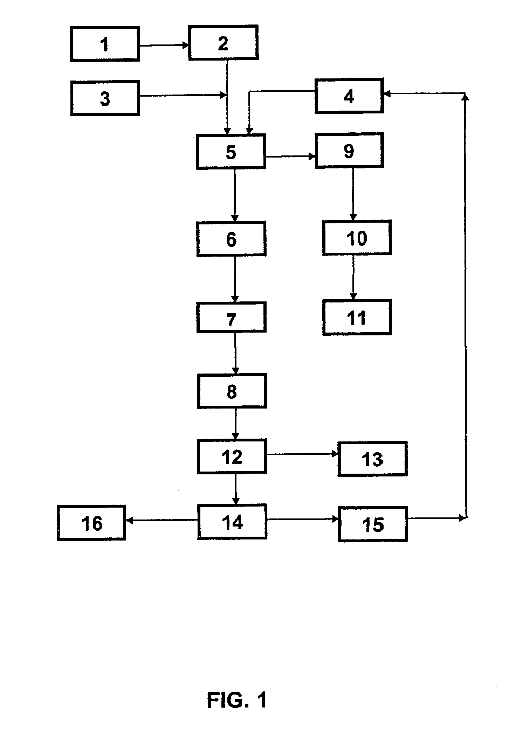

For better comprehension of the invention, the description thereof shall be made in conjunction with Figure 1 accompanying the present specification, forming an integral part thereof.

### Preferred method of embodiment

As schematically illustrated in Figure 1, the hydroxylated fatty esters generated in the production of biodiesel from castor bean oil (1) are subjected to a nitration stage at (2) with a mixture of acetic anhydride (or any other nitration agent) and nitric acid or any other nitrating mixture. The mass ratio between the nitric acid and the nitrating mixture may lie between 0.05 and 1.00 and the concentration of nitric acid may lie between 5% and 70% (w/w).

Other nitrating mixtures may also be employed (for example nitric acid in a sulphuric medium, etc), taking into account their specific composition and conditions of reaction applicability.

During the stage of nitration of the hydroxyl site there may occur on a smaller scale the nitration of double bonds or even of saturated carbons.

The temperature of reaction of nitration of the hydroxyl site influences the process and lies between 0 ° and 50 °C, preferably between 0 ° and 25 °C, more preferably between 0 ° and 15 °C, it being observed that the lower the temperature at which the reaction of nitration of the hydroxyl site occurs the more selective the process is.

The time of reaction of nitration of the hydroxyl site also strongly influences the process and may lie between 1 and 60 minutes, preferably between 20 and 60 minutes, more preferably between 40 and 60 minutes.

After the time determined for reaction to occur water (3) is added to the reaction mixture and the temperature is reduced to approximately 0 °C.

Subsequently the mixture receives the addition of ethyl ether (4) to promote phase separation (5) wherein the nitrating mixture (9) is separated and which continues for treatment (10) and discarding (11), and the crude nitrated additive (6) continues for consecutive washings (7) with water until achievement of a pH of approximately 7 in the intermediate product, being the hydrated nitrated additive (8).

The resulting mixture is passed through a dryer separator (12) comprising a drying agent (for example calcium chloride) wherein residual water is removed (13).

The resulting liquid phase composed of anhydrous nitrated additive and ethyl ether passes through an evaporator (14) for the purpose of removal of the ether (15) and obtainment of the pure nitrated additive (16).

### Representative example

The representative example given below is of a purely illustrative nature and must not be considered in any manner as limiting the invention.

Typically, values of diesel cetane numbers lie between 42 and 47 and normally a cetane improvement additive considered as being effective will increase said cetane number by approximately 2 points at concentrations of application in quantities of approximately 250 mg/l in the stream of diesel.

ASTM standards require the use of specific apparatus to determine the cetane number of a fuel, known as an Ignition Quality Tester (IQT).

In the representative example provided below the efficacy of the cetane improvement additive produced by the present invention is evaluated employing the cetane index measured resulting from application of said additive to a diesel oil, denominated standard diesel, utilising 3 different concentrations (500 mg/l, 1000 mg/l and 1500 mg/l) of said cetane improvement additive to such diesel oil, in comparison with the cetane index measured on the said diesel oil utilising a concentration of 500 mg/l of the imported commercial additive 2-ethylhexyl nitrate (2-EHN).

For the preparation of such representative example castor bean biodiesel (20 ml) containing 80% ricinoleic acid was nitrated with a previously-prepared mixture (114 ml) containing 72% (w/w) acetic anhydride and 28% (w/w) 70% nitric acid in a jacketed reactor with magnetic stirring. The temperature during preparation of said mixture and during the nitration reaction was maintained at 15 °C.

After 60 minutes 400 ml of water and ice was added to the reaction mixture for the purpose of immediately interrupting the reaction. Diethyl ether was added to promote phase separation. The aqueous phase generated was separated off, successive washes with water being required to attain a neutral pH for the same.

Following the washing process the additive generated was dried with a drying agent (calcium chloride) and the ether was removed by evaporation.

The nitrated additive produced by the present invention synthesised at 15 °C was mixed into the diesel and tested by the IQT method (Ignition Quality Tester - ASTM D-6890).

**For comparison purposes there were tested:**

| | |
|---|---|
| 1 | Pure diesel (with no additive); |
| 2 | Diesel + 2-EHN additive (commercial) at an addition rate of 500 mg/l; |
| 3 | Diesel + cetane improvement additive of the present invention, at addition rates of 500 mg/l, 1000 mg/l, 1500 mg/l. |

The result of such tests in terms of cetane index utilising the synthesised additive of the present invention as described in the Example are shown in Table 1, providing comparative data of variations in cetane number obtained by the addition of 500 mg/l of the additive most-commonly utilised as cetane improver, 2-ethylhexyl nitrate (2-EHN), in relation to the additive of the present invention produced from nitration of biodiesel derived from castor bean oil at addition concentrations of 500 mg/l, 1000 mg/l and 1500 mg/l, such cetane index evaluations being obtained by ASTM D-6890 methodology, denominated Ignition Quality Test (IQT).

| TABLE 1 | |
|---|---|
| Product | DCN (§) |
| Standard diesel | 46.0 |
| Standard diesel + 2-EHN (500 mg/l) | 48.8 |
| Standard diesel + additive (500 mg/l) | 47.9 |
| Standard diesel + additive (1000 mg/l) | 49.0 |
| Standard diesel + additive (1500 mg/l) | 50.3 |
| *§: DCN* = *diesel cetane number* | |

From the results provided in Table 1 it may be observed that the cetane improvement additive of the present invention yielded cetane index values lying within the usual range of acceptability for said parameter, in comparison with those usually obtained through utilisation of the commercial 2-EHN additive. From the data it is estimated that to obtain the same cetane increase achieved through the addition of 500 mg/l of 2-ethylhexyl nitrate, approximately 900 mg/l of the additive of the present invention requires to be added. However, even in view of such a possible increase in the quantity of addition in respect of the additive of the present invention to obtain results equivalent to those described for the commercial 2-EHN additive, such additive of the present invention represents a very significant alternative by virtue of having a cost very much lower than that of the commercial product offered, in addition to the aforesaid advantages.

All references herein mentioned have been introduced in their totality. Although the present invention has been described by means of its preferential method of embodiment and representative example the principal concept underlying the present invention, being that of direct nitration at the site of the hydroxyl functionality already present in the natural chemistry of the molecule of the fatty acid ester generated in the production of biodiesel produced from castor bean oil, is asserted in terms of its innovative nature, in relation whereto those usually versed in the art will be capable of discerning and making appropriate variations, modifications, alterations, adaptations and equivalents compatible with the working environment in question without however departing from the comprehensiveness of the spirit and scope of the present invention, represented by the claims which follow.

## Claims

1. Method for production of cetane improvement additive **characterised by** comprising nitration of a hydroxylated fatty acid deriving from a triglyceride of vegetable origin possessing a hydroxyl functionality in its molecular chemical structure.

2. Method according to claim 1 in which the triglyceride of vegetable origin comprises castor bean oil.

3. Method according to claim 1 **characterised by** nitration of a naturally-hydroxylated fatty ester comprising castor bean biodiesel.

4. Method according to claim 1, 2 or 3 in which the nitration occurs directly at the site of the hydroxyl functionality.

5. Method according to any preceding claim in which the reaction temperature of the nitration is from 0 ° to 50 °C, preferably from 0 ° to 15 °C.

6. Method according to any preceding claim in which the reaction time of the nitration is from 1 to 60 minutes, preferably from 40 to 60 minutes.

7. Method for production of cetane improvement additive **characterised by** comprising nitration of a naturally-hydroxylated fatty acid deriving from a triglyceride of vegetable origin already possessing a hydroxyl functionality in its natural molecular chemical structure.
